# EUROPEAN PATENT SPECIFICATION

(11) **EP 2 666 498 B1**
(45) Date of publication and mention of the grant of the patent: **25.11.2020**
(21) Application number: 13169192.5
(22) Date of filing: 24.05.2013
(51) Int. Cl.: A61M 15/00

(54) **Inhaler device having an improved guide gear**
Inhalatorvorrichtung mit verbesserter Führungsausrüstung
Dispositif inhalateur comportant une roue de guidage améliorée

(30) Priority: 25.05.2012 TR 201206167; 08.02.2013 TR 201301562
(43) Date of publication of application: 27.11.2013
(73) Proprietor: Arven Ilac Sanayi Ve Ticaret A.S., Istanbul 34460 (TR)
(72) Inventor: Toksöz, Zafer, 34460 Istanbul (TR); Cifter, Ümit, 34460 Istanbul (TR); Türkyilmaz, Ali, 34460 Istanbul (TR); Mutlu, Onur, 34460 Istanbul (TR)
(74) Representative: Mutlu, Aydin

(56) References cited:
- EP-A1- 2 239 001
- US-A1- 2010 307 491
- US-A1- 2011 259 328

## Description

### Field of Invention

The present invention relates to improvements carried out in the gear members of inhaler devices enabling the administration of dry powder inhalation drugs.

### Prior Art

Diseases such as asthma, bronchitis, and COLD (Chronic Obstructive Lung Disease) substantially decrease the quality of human life, despite the developments which have been made in the diagnosis and therapy thereof in the recent years. It has been proposed to administer medicaments via inhalers for optimizing the treatment of such diseases. The inhaler route of treatment is the most preferred one and it is expected to remain so, as the first option, in the future. The most important advantage of using medicaments via inhalation is based on providing a more efficient therapy by making use of lower amounts of medicaments, delivering higher concentrations of medicaments to the airways, and particularly decreasing the systemic side effects of the medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

Nowadays, there have been developed various inhalation devices for administering inhalation medicaments. These devices are basically classified into two groups, i.e. metered dose inhalers and dry powder inhalers. These types of devices are structurally provided with basic components such as an actuator, counter, body, lid, lock, etc. Additionally, powder inhalation medicaments are kept in carriers such as blisters, capsules, etc. Blisters are composed of two basic parts, a main layer provided with cavities carrying the medicament, and a strippable protective layer.

Such type of inhaler devices having multiple blisters comprise various actuator and force transmission members. These members are generally articulated to each other and are disposed in a protective body. The body typically has a complementing two-part structure. One of these parts is positioned below said members to provide a basis, whereas the other one is positioned above these members in the form of a cover. Actuator and force transmission members disposed between the body parts are designed to provide the best use of a drug-carrying blister. The actuator and force transmission members comprise gears of various types and sizes. A congruent operation of these gears makes it possible to administer a drug. Said gears may become damaged due to jams occurring during use, or cannot be run due to the inability to advance the blister. This case is caused with the drug in the blister opened for administration pouring down into the device under the influence of the actuating parts and of the air, and accumulating between the upper surface of the gears and the part of the body just on the top of these components. In other words, the powder which is poured from the blister jams the mechanism by getting deposited between the upper surface of the gears and the inner part of the body. On the other hand, the multipart structure of the mechanism brings about a high size requirement and this, in turn, causes inconvenience for users in terms of carrying and using the device. For this reason, it turns out to be a necessity to use a minimal size for the body and the inner mechanism. When the size of the body is minimum, this makes the distance between the members of the inner mechanism and the protective body too small and therefore any powder drug residues accumulating therein jam the mechanism. Such mechanisms in which these problems may be encountered can be found in the applications and patents EP1292510 B1, WO03/035508 A1, EP1436217.

The complicated multipart structure of many of the blister advancement mechanisms and the problems concerning the placement of the members leads to application- and use-related difficulties and prevent the users from obtaining maximum efficiency.

In result, there is a novelty required in the field of inhaler devices, providing high-accuracy operation, as well as advantages in terms of device size and use.

### Object and Brief Description of Invention

The present invention relates to a novel gearing for an inhaler device used for dry powder inhalation, eliminating all problems mentioned above and bringing additional advantages to the relevant prior art.

EP 2 239 001 A1 discloses a dry powder inhaler device having an advancing a blister with a plurality of medicament-carrying cavities in a single direction, said blister being placed in the interior of an outer housing and guided to an inner body from one side, and from the other side to a mouthpiece, said device further comprising an actuating element slid into the interior of said outer housing and a lid connected onto the outer housing, wherein said device further comprises a locking clips containing at least one lug retaining said actuating element at a certain displaced distance to which it is pushed, and connected to the outer body in a way that allows said clips to be moved vertically up- and downward, so as to ensure that each cavity of which the turn comes for use can be completely peeled off.

Accordingly, the main object of the present invention is to provide an accurately-operating blister advancement mechanism for use in a dry powder inhaler device.

Another object of the present invention is to provide a gear embodiment enabling to use a minimum size.

A further object of the present invention is to provide a smoothly-operating blister advancement mechanism by virtue of gearing members which prevent the occurrence of jamming and breaking.

In order to achieve all objects described above and to emerge from the following detailed description, a dry powder inhaler device has been developed which comprises an outer body, an advancement mechanism housed in an inner part of the outer body, and a guide gear in this mechanism, said guide gear having a body provided with grooves into which blister cavities filled with a powder drug are placed.

In a preferred embodiment, said novelty according to the present invention is based on providing at least one pocket formed on an upper surface of the guide gear, said pocket enabling to release any powder drug accumulated therein.

In another preferred embodiment according to the present invention, one end of said pocket is opened to an inner edge of the guide gear.

In a further preferred embodiment according to the present invention, one end of said pocket is opened to an outer edge of the guide gear.

In another preferred embodiment according to the present invention, the end of said pocket is opened to the inner edge and the outer edge of the guide gear.

In a further preferred embodiment according to the present invention, the base of the pocket is inclined towards the end part.

In another preferred embodiment according to the present invention, the basal surface of the pocket is in the form of a square, rectangle, ellipse, circle or triangle.

### Brief Description of Figures

Figure 1 is an illustration of a representative embodiment according to the present invention.
Figure 2 is an exemplary illustration of the components making up the outer body of the device according to the present invention.
Figure 3 is an exemplary illustration of an advancement mechanism and the inner part of the device body according to the present invention.
Figure 4 is an exemplary illustration of the advancement mechanism and the inner part of the device body according to the present invention.
Figure 5 is an exemplary illustration of a guide gear according to the present invention.
Figure 6 is an exemplary illustration of the guide gear according to the present invention.
Figure 7 is an exemplary illustration of the guide gear and a pocket according to the present invention.
Figure 8 is an exemplary illustration of a blister and cavities according to the present invention.

### Reference Numbers in Figures

- 1.: Outer body
- 2.: Cavity groove of the guide gear
- 3.: Blister
- 4.: Blister cavity
- 5.: Guide gear
- 6.: Inhaler device
- 7.: Upper surface of the guide gear
- 8.: Pocket
- 9.: Edge of the pocket
- 10.: Inner edge of the upper surface of the guide gear
- 11.: Outer edge of the upper surface of the guide gear
- 12.: Base of the pocket
- 13.: Mouthpiece
- 14.: Body part
- 15.: Body part

### Detailed Description of Invention

In the following detailed description, an inhaler device (6) according to the present invention shall be described illustratively by making references to the accompanying figures, only to make it clear without imposing any restrictions thereon.

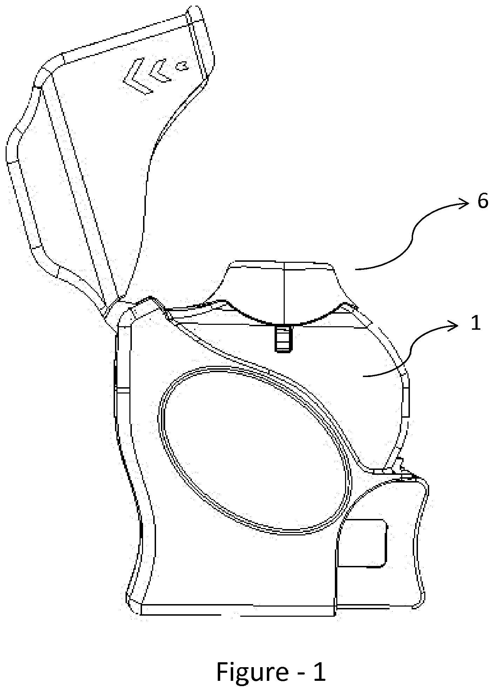

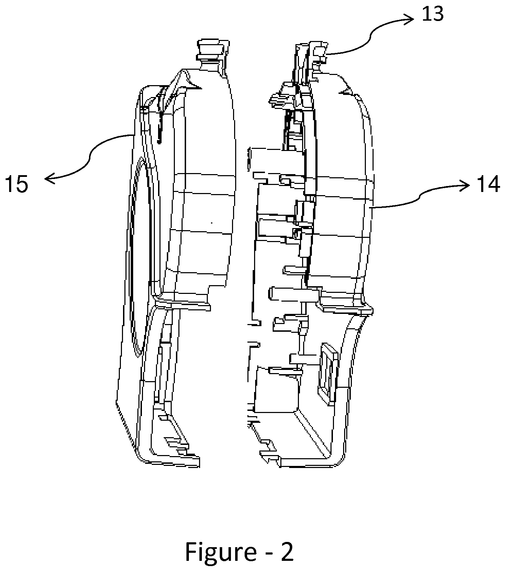

An outer body (1) of the inhaler device (6) according to the present invention, of which an exemplary illustration is given in figures 1 and 2, is composed of two complementary parts (14, 15), provided with retaining tabs at their periphery. The outer body (1) is made by assembling these two parts to each other. The upper part of the outer body (1) is provided with a mouthpiece (13) enabling a user to inhale a medicament. Furthermore, a lid (7) is provided, which preferably can be rotated as a result of being pivoted to the outer body (1) and can be closed to cover the mouthpiece in order to keep clean the mouthpiece when the inhaler device (6) is not in use and to prevent any unwanted substances from entering into the device.

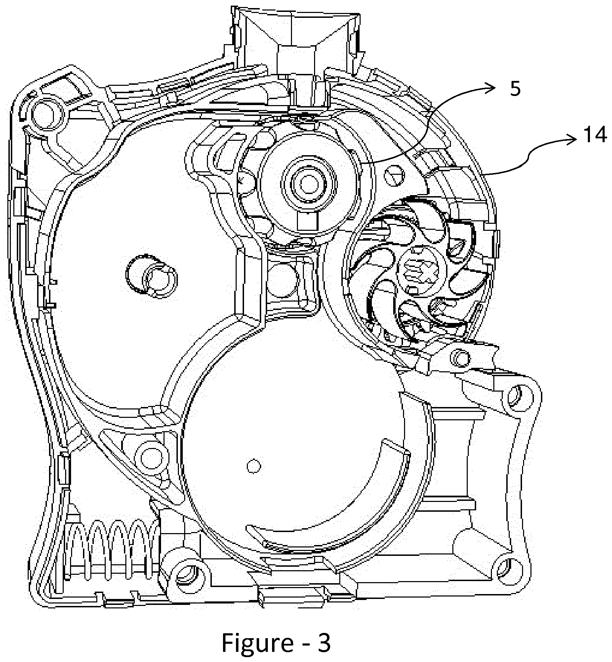

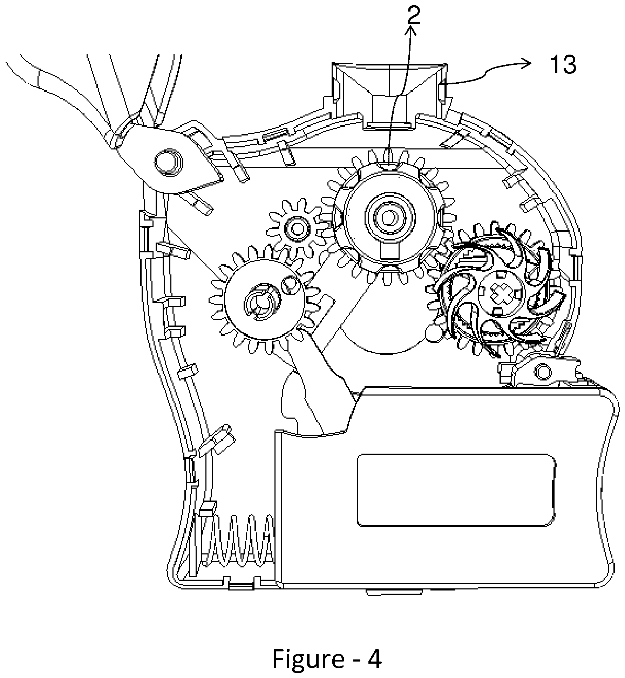

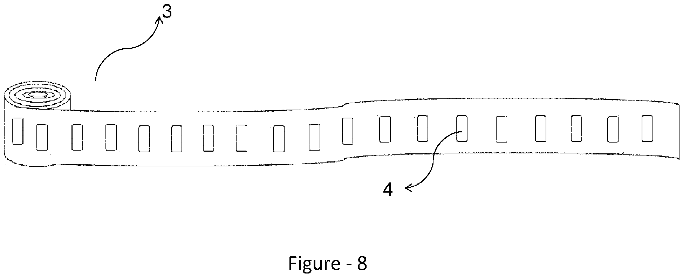

According to the exemplary illustrations given in figures 1, 3, 4, 5, and 8, a gear set of the advancement mechanism is disposed in an inner part of the outer body (1) to provide the operation of the inhaler device (6). Along some intermediary channels of this gear set are provided a blister (3) having cavities (4) filled with a powder drug. The gear set further enables to move the blister in one direction only. This gear set comprises a guide gear (5) just below the mouthpiece, the body of this guide gear having grooves (2) into which the blister (3) cavities (4) containing a powder drug is placed. The grooves (2) of the guide gear are positioned exactly below the mouthpiece of the inhaler device.

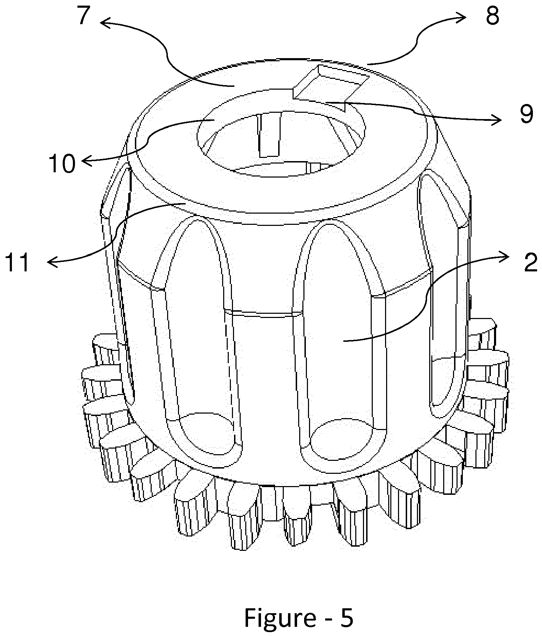

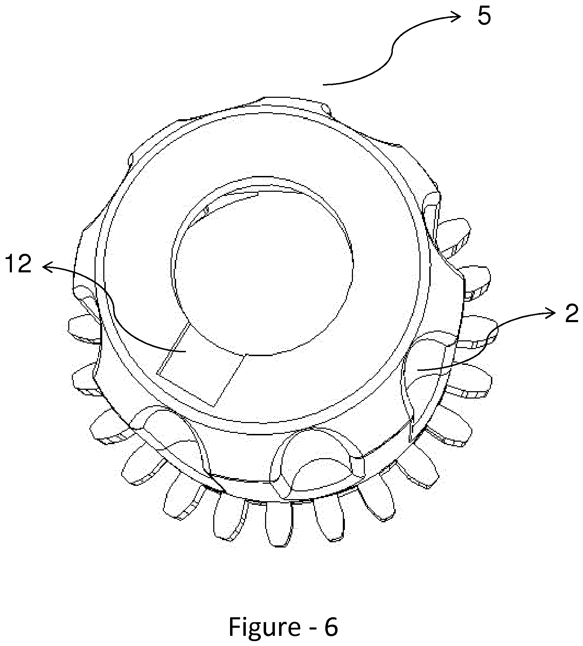

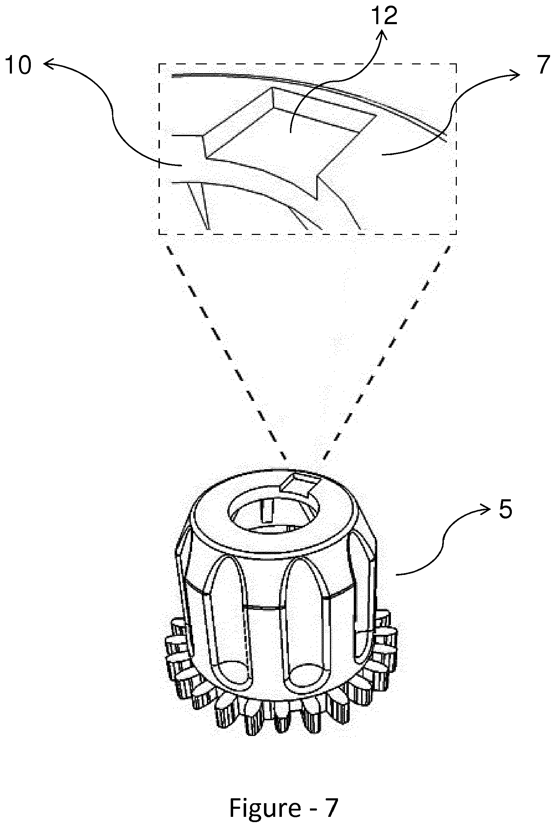

According to the exemplary illustrations shown in figures 5, 6, and 7, the guide gear (5) is provided with grooves (2) thereon and the teeth of the gear are aligned along the basal periphery thereof. A planar surface (7) is provided at the upper part of the guide gear. A pocket (8) is formed in this upper surface (7). One edge (9) of the pocket (8) can be opened to an inner edge (10) or to an outer edge (11), or to both of these edges of the guide gear (5). The base of this pocket (8) can have a straight, a sunken, or a stepwise surface structure. The base (12) of the pocket is preferably formed in an inclined manner towards the inner edge or the outer edge of the pocket. The surface of the pocket base (12) can have a square, rectangular, oval, circular, or a triangular form, or any other forms.

An unused filled blister (3) is extended along the intermediate channels and separated into two parts, i.e. the main layer and the protective layer, by means of the mechanism. The grooves of the guide gear is positioned exactly below the device mouthpiece such that a drug-filled cavity (4), which is first in the queue, is administered. During administration, even a trace amount of a powder drug can be jammed between the upper surface (7) of the guide gear (5) and the inner surface of the outer body. This jamming prevents the motion of the entire advancement mechanism to such an extent that if the device is forced to operate, the mechanism breaks down. In order to overcome this problem, a pocket (8) is formed on the planar surface (7) disposed on the upper part of the guide gear (5). Any powders getting jammed between the upper surface (7) and the inner part of the outer body (1) are poured into this pocket (8) with the scraping action of the drug during operation, and are removed from the pocket to other parts which would not block the mechanism. By virtue of the pocket (8), the jamming of powders is prevented and the distance between the body (1) and the upper surface (7) of the guide gear (5) is minimized. In result, a device embodiment is achieved having a smoothly-operating blister advancement mechanism and is advantageous in terms of size.

## Claims

1. A dry powder inhaler device (6) comprising an outer body (1), an advancement mechanism housed in an inner part of the outer body, and a guide gear (5) in this mechanism, said guide gear having a body provided with grooves (2) into which blister (3) cavities (4) filled with a powder drug are placed, **characterized in that**
- a planar surface (7) is provided at the upper part of the guide gear (5),
- said guide gear (5) comprises at least one pocket (8) formed on the planar surface (7) of the guide gear, one edge (9) of the pocket (8) being open to at least one of the inner (10) or outer (11) edges of the guide gear, said pocket (8) enabling to release or remove any powder drug accumulated therein, such that, during operation, the powder drug jammed between the upper surface (7) of the guide gear (5) and the inner surface of the outer body (1) is removed.

2. The dry powder inhaler device (6) according to claim 1 wherein the at least one pocket (8) has a base (12) inclined towards the inner or the outer edge of the pocket.

3. The dry powder inhaler device (6) according to any of the preceding claims, wherein the surface of the base (12) of the pocket has a square, rectangular, oval, circular, or triangular shape.

## Patentansprüche

1. Trockenpulver-Inhalationsvorrichtung (6), umfassend einen Außenkörper (1), einen Vorschubmechanismus, der in einem inneren Teil des Außenkörpers untergebracht ist, und ein Führungszahnrad (5) in diesem Mechanismus, wobei das Führungszahnrad einen Körper aufweist, der mit Einkerbungen (2) versehen ist, in die mit einem Pulverarzneimittel gefüllte Hohlräume (4) eines Blisters (3) eingebracht sind, **dadurch gekennzeichnet, dass**
- eine planare Fläche (7) an dem oberen Teil des Führungszahnrads (5) vorgesehen ist,
- das Führungszahnrad (5) mindestens eine Tasche (8) umfasst, die auf der planaren Fläche (7) des Führungszahnrads ausgebildet ist, wobei ein Rand (9) der Tasche (8) zu zumindest einem von den inneren (10) oder äußeren (11) Rändern des Führungszahnrads offen ist,
wobei die Tasche (8) es ermöglicht, darin angesammeltes Pulverarzneimittel freizusetzen oder zu entfernen, sodass das zwischen der oberen Fläche (7) des Führungszahnrads (5) und der inneren Oberfläche des Außenkörpers (1) eingeklemmte Pulverarzneimittel während des Betriebes entfernt wird.

2. Trockenpulver-Inhalationsvorrichtung (6) nach Anspruch 1, wobei die zumindest eine Tasche (8) einen Boden (12) aufweist, der zu dem inneren oder dem äußeren Rand der Tasche hin geneigt ist.

3. Trockenpulver-Inhalationsvorrichtung (6) nach einem der voranstehenden Ansprüche, wobei die Fläche des Bodens (12) der Tasche eine quadratische, rechteckige, ovale, kreisförmige oder dreieckige Form aufweist.

## Revendications

1. Dispositif inhalateur de poudre sèche (6) comprenant un corps externe (1), un mécanisme d'avance logé dans une partie interne du corps externe, et une roue de guidage (5) prévue dans ce mécanisme, ladite roue de guidage ayant un corps muni de rainures (2) dans lesquelles sont placées des cavités (4) de plaquettes thermoformées (3) remplies d'un médicament en poudre, **caractérisé en ce que** :
- une surface plane (7) est placée au niveau de la partie supérieure de la roue de guidage (5),
- ladite roue de guidage (5) comporte au moins une poche (8) formée sur la surface plane (7) de la roue de guidage, un bord (9) de la poche (8) étant ouvert sur au moins l'un des bords intérieur (10) ou extérieur (11) de la roue de guidage,
ladite poche (8) permettant de libérer ou de retirer tout médicament en poudre qui s'y est accumulé, de sorte que, lors de l'opération, le médicament en poudre bloqué entre la surface supérieure (7) de la roue de guidage (5) et la surface intérieure du corps externe (1) est retiré.

2. Dispositif inhalateur de poudre sèche (6) selon la revendication 1, dans lequel la au moins une poche (8) comporte une base (12) inclinée vers le bord intérieur ou le bord extérieur de la poche.

3. Dispositif inhalateur de poudre sèche (6) selon l'une quelconque des précédentes revendications, dans lequel la surface de la base (12) de la poche a une forme carrée, rectangulaire, ovale, circulaire ou triangulaire.
